# EUROPÄISCHE PATENTSCHRIFT

(11) **EP 4 037 729 B1**
(45) Veröffentlichungstag und Bekanntmachung des Hinweises auf die Patenterteilung: **19.08.2026**
(21) Anmeldenummer: 20780181.2
(22) Anmeldetag: 24.09.2020
(51) Int. Cl.: A61M 5/14, A61M 5/40, A61M 5/168

(54) **TROPFKAMMERANORDNUNG FÜR EIN MEDIZINISCHES INFUSIONSSYSTEM**
DRIP CHAMBER ASSEMBLY FOR A MEDICAL INFUSION SYSTEM
ENSEMBLE CHAMBRE DE GOUTTE-À-GOUTTE POUR UN SYSTÈME DE PERFUSION MÉDICAL

(30) Priorität: 30.09.2019 DE 102019215022
(43) Veröffentlichungstag der Anmeldung: 10.08.2022
(73) Patentinhaber: B. Braun Melsungen AG, 34212 Melsungen (DE)
(72) Erfinder: SCHLITT, Christof, 34621 Obergrenzebach (DE)
(74) Vertreter: Patentanwälte Ruff, Wilhelm, Beier, Dauster & Partner mbB
(86) Internationale Anmeldenummer: PCT/EP2020/076708
(87) Internationale Veröffentlichungsnummer: WO 2021/063797

(56) Entgegenhaltungen:
- WO-A1-2004/064904
- WO-A1-2012/057602
- DE-A1- 3 800 319
- US-A- 4 096 879
- US-A1- 2015 374 909
- US-B1- 6 213 986

## Beschreibung

Die Erfindung betrifft eine Tropfkammeranordnung mit den Merkmalen des Oberbegriffs des Anspruchs 1.

Aus der DE 38 00 319 A1 ist eine Tropfkammeranordnung mit den oberbegrifflichen Merkmalen des Anspruchs 1 bekannt. Die bekannte Tropfkammeranordnung weist ein schwimmfähiges Ventil auf, das durch ein blickdurchlässiges Tropfkammergehäuse hindurch einsehbar ist und grundsätzlich die Kontrolle eines in dem Tropfkammergehäuse vorhandenen Flüssigkeitspegels erlaubt. Das Ventil ist extrem weich und damit besonders formnachgiebig. Steigt der Flüssigkeitspegel, schwimmt das Ventil auf und gelangt in einen Bereich oberhalb eines Pumpabschnitts des Tropfkammergehäuses.

Weiter ist aus der WO 2012/057602 A1 eine Tropfkammeranordnung mit einem als Auftriebskörper gestalteten Ventilkörper bekannt, der in einem Tropfkammergehäuse schwimmbeweglich ist. Das Tropfkammergehäuse selbst ist nicht pumpbeweglich. Stattdessen ist ein zusammendrückbarer Körper vorgesehen, der fluidleitend mit dem Tropfkammergehäuse verbunden und pumpbeweglich ist. Der zusammendrückbare Körper ist abseits des Tropfkammergehäuses angeordnet. Der Ventilkörper kann deshalb weder durch den zusammendrückbaren Körper taktil wahrgenommen werden noch dessen Pumpbeweglichkeit verringern.

Eine weitere Tropfkammeranordnung ist im Bereich der Medizintechnik allgemein bekannt und als Komponente eines Infusionssystems, das auch als Überleitungssystem bezeichnet werden kann, zur Verwendung bei einer Infusionstherapie vorgesehen. Die allgemein bekannte Tropfkammeranordnung weist ein Gehäuse auf, das eine Tropfkammer zur Aufnahme einer zu verabreichenden medizinischen Flüssigkeit umgrenzt. Das Gehäuse ist mit einem Einlass und mit einem Auslass versehen. Der Einlass ist zum tropfenweisen Einleiten in und der Auslass zu einem Ableiten der medizinischen Flüssigkeit aus der Tropfkammer vorgesehen. Bei der Infusionstherapie stellt sich ein gewisser Flüssigkeitspegel der medizinischen Flüssigkeit in der Tropfkammer ein, der weder zu hoch noch zu niedrig sein soll. Ein zu niedriger Flüssigkeitspegel kann dazu führen, dass durch den Einlass in die Tropfkammer einfallende Tropfen Luft in eine der Tropfkammer nachgelagerte Schlauchleitung des Infusionssystems spülen. Ein zu hoher Flüssigkeitspegel kann dazu führen, dass sogenannte Keimstraßen entstehen. Beides ist einer Patientensicherheit abträglich. Zur visuellen Kontrolle des Flüssigkeitspegels ist das Gehäuse der bekannten Tropfkammeranordnung daher aus blickdurchlässigem Kunststoff ausgebildet. Zur verbesserten visuellen Kontrolle weist die bekannte Tropfkammeranordnung einen Pegelindikator in Form eines statischen Füllstrichs auf, der auf eine Wandung des blickdurchlässigen Gehäuses aufgebracht ist.

Weiter ist aus der US 6 213 986 B1 eine Tropfkammeranordnung mit einer ersten Fluidkammer und einer zweiten Fluidkammer bekannt, wobei in der ersten Fluidkammer ein Schwimmventil angeordnet ist.

Weiter ist aus der US 4 096 879 A eine Vorrichtung zur Flussregelung bekannt, die eine obere Fluidkammer und eine separate untere Fluidkammer aufweist. Die obere Fluidkammer weist ein Schwimmventil auf, das in Abhängigkeit eines Flüssigkeitspegels entweder einen Einlass oder einen Auslass der oberen Fluidkammer verschließt bzw. freigibt.

Weiter ist aus der WO 2004/064904 A1 ein Infusionsset mit einer Fluidkammer und einem Schwimmer bekannt, wobei der Schwimmer in der Fluidkammer angeordnet ist.

Weiter ist aus der US 2015/374909 A1 eine Tropfkammeranordnung mit einem Schwimmer zur Flussregelung bekannt.

Aufgabe der Erfindung ist es, eine Tropfkammeranordnung der eingangs genannten Art bereitzustellen, die gegenüber dem Stand der Technik eine verbesserte Patientensicherheit ermöglicht.

Diese Aufgabe wird dadurch gelöst, dass der Pegelindikator ein in der Tropfkammer beweglich angeordnetes Funktionselement ist, das auf der medizinischen Flüssigkeit schwimmfähig ausgebildet ist und in Abhängigkeit des Flüssigkeitspegels in Axialrichtung der Tropfkammer zwischen unterschiedlichen Schwimmpositionen verlagerbar ist und wenigstens in einer Pegelschwimmposition, die einem angestrebten Flüssigkeitspegel zugeordnet ist, zur Kontrolle des Flüssigkeitspegels durch das wenigstens abschnittsweise blickdurchlässige Gehäuse hindurch einsehbar ist. Durch die erfindungsgemäße Lösung ist der Flüssigkeitspegel insbesondere bei einem initialen Befüllen der Tropfkammer mit medizinischer Flüssigkeit zum Ingangsetzen der Infusion in deutlich verbesserter Weise visuell kontrollierbar. Denn bei einem solchen initialen Befüllen verändert das Funktionselement seine Schwimmposition in der Tropfkammer. Diese Veränderung ist von medizinischem Personal vergleichsweise einfach und eindeutig wahrnehmbar. Insbesondere gegenüber im Stand der Technik bekannten Tropfkammeranordnungen mit Füllstrichen zur Kontrolle des Flüssigkeitspegels ergeben sich daher deutliche Vorteile. Denn durch die erfindungsgemäße Lösung kann auf einen visuellen Abgleich zwischen einem tatsächlichen Flüssigkeitspegel und dem Füllstrich verzichtet werden. Eine solche Kontrolle ist vergleichsweise zeitaufwändig, unterliegt einer fehlerträchtigen Parallaxe und ist insbesondere aus der Ferne schwer durchführbar. Das Funktionselement ist schwimmfähig ausgebildet, wobei eine Dichte des Funktionselements dementsprechend auf eine Dichte der zu verabreichenden medizinischen Flüssigkeit abgestimmt ist. Um die Schwimmfähigkeit des Funktionselements für möglichst viele unterschiedliche medizinische Flüssigkeiten zu gewährleisten, ist es vorteilhaft, wenn die Dichte des Funktionselements geringer ist als die Dichte von Wasser. Das Funktionselement ist vorzugsweise aus einem Kunststoff gefertigt. Das Funktionselement kann als massives Strukturelement ausgebildet sein. Alternativ kann das Funktionselement eine Werkstoffstruktur aufweisen, die einen Auftrieb erzeugt und/oder unterstützt, beispielsweise eine Schaum-, Waben- und/oder Porenstruktur. Das Funktionselement ist in Axialrichtung der Tropfkammer frei schwimmbeweglich. Insoweit ist ein radialer Außendurchmesser des Funktionselements auf einen radialen Innendurchmesser der Tropfkammer abgestimmt und geringer als dieser, so dass die freie Schwimmbeweglichkeit des Funktionselements in Axialrichtung gewährleistet ist. Die Axialrichtung der Tropfkammer ist in einem Gebrauchszustand der Tropfkammeranordnung parallel zu einer Fallrichtung von in die Tropfkammer eintropfender medizinsicher Flüssigkeit orientiert. Das Gehäuse kann ein- oder mehrteilig ausgebildet sein. Vorzugsweise ist das Gehäuse ein wenigstens zweiteiliges Gehäuse und weist dementsprechend ein Tropfkammeroberteil und ein Tropfkammerunterteil auf. Sofern eine solche Gestaltung vorgesehen ist, ist es vorteilhaft, wenn das Funktionselement beweglich in dem Tropfkammerunterteil angeordnet ist.

In Ausgestaltung der Erfindung ist das Funktionselement in allen Schwimmpositionen einsehbar. D.h. das Funktionselement ist unabhängig von dem Flüssigkeitspegel stets einsehbar, insbesondere auch in einem nicht mit Flüssigkeit befüllten Zustand der Tropfkammer. Hierdurch kann eine nochmals verbesserte visuelle Kontrolle des Flüssigkeitspegels erreicht werden. Das Gehäuse ist dementsprechend blickdurchlässig gestaltet, so dass das Funktionselement in sämtlichen in der Tropfkammer einnehmbaren Positionen einsehbar ist.

In weiterer Ausgestaltung der Erfindung weist das Funktionselement eine Dichte auf, die kleiner als 1,05 g/cm³, bevorzugt kleiner als 0,50 g/cm³, und besonders bevorzugt kleiner als 0,10 g/cm³ ist. Sofern die Dichte des Funktionselements kleiner als 1,05 g/cm³ ist, ist jedenfalls eine Schwimmfähigkeit auf isotonischer Kochsalzlösung gewährleistet, was für einige Anwendungsfälle der Tropfkammeranordnung ausreichend sein kann. Um die Schwimmfähigkeit des Funktionselements für sämtliche üblicherweise zur Verabreichung relevanten und/oder infrage kommenden medizinischen Flüssigkeiten zu gewährleisten, ist es besonders vorteilhaft, wenn die Dichte des Funktionselements kleiner 0,50 g/cm³ ist. Bei einer Dichte des Funktionselements, die kleiner als 0,10 g/cm³ ist, ist besonders bevorzugt gewährleistet, dass das Funktionselement besonders leichtgängig aufschwimmt. Dies ist insbesondere zur visuellen Kontrolle des Flüssigkeitspegels bei einem initialen Befüllen der Tropfkammer mit medizinischer Flüssigkeit von Vorteil. Die Dichte des Funktionselements ist nicht notwendigerweise identisch mit einer Werkstoffdichte eines Werkstoffs, aus dem das Funktionselement gefertigt ist. Dies insbesondere dann, wenn das Funktionselement mit wenigstens einem Hohlraum, einer Poren-, Waben- und/oder Schraubenstruktur versehen ist.

Weiter gemäß der Erfindung weist das Gehäuse einen Pumpabschnitt auf, der in Radialrichtung der Tropfkammer elastisch formnachgiebig ausgebildet und in Axialrichtung der Tropfkammer zwischen dem Einlass und dem Auslass angeordnet ist, wobei der Pumpabschnitt zum initialen Ansaugen der medizinischen Flüssigkeit durch den Einlass mittels einer manuellen radialen Pumpbewegung zwischen einem in Radialrichtung eingedrückten Pumpzustand und einem nicht manuell eingedrückten Ruhezustand überführbar ist, und wobei das Funktionselement im Vergleich zu dem Pumpabschnitt formstabil ausgebildet und in der Pegelschwimmposition im eingedrückten Pumpzustand des Pumpabschnitts durch den Pumpabschnitt hindurch taktil wahrnehmbar und in einer weiteren Schwimmposition, die einem niedrigeren Flüssigkeitspegel zugeordnet ist, taktil nicht wahrnehmbar ist. Durch diese Ausgestaltung der Erfindung erhält eine die Tropfkammeranordnung handhabende Person eine taktile Rückmeldung beim initialen Befüllen der Tropfkammer bzw. beim Starten der Infusion, sobald die Pegelschwimmposition und damit der angestrebte Flüssigkeitspegel in der Tropfkammer erreicht ist. Hierdurch wird eine nochmals verbesserte Patientensicherheit erreicht. Der Pumpabschnitt dient einem Ingangsetzen einer zu verabreichenden Infusion. Zu diesem Zweck kann der Pumpabschnitt zum Ansaugen der medizinischen Flüssigkeit durch den Einlass manuell in Radialrichtung der Tropfkammer elastisch eingedrückt werden. Durch eine solche manuelle Pumpbewegung wird der Pumpabschnitt zwischen dem eingedrückten Pumpzustand und dem nicht eingedrückten Ruhezustand verlagert. Diese manuelle Pumpbewegung kann so lange wiederholt werden, bis der angestrebte Flüssigkeitspegel erreicht ist. Da das Funktionselement in der Pegelschwimmposition, die dem angestrebten Flüssigkeitspegel zugeordnet ist, taktil wahrnehmbar ist, erhält die Bedienperson eine unmittelbare Rückmeldung. Hierdurch wird ein unbeabsichtigtes Überfüllen oder Unterfüllen der Tropfkammer bei Ingangsetzen der Infusion vermieden. Dies ist eine besonders vorteilhafte Ausgestaltung der Erfindung. Um die taktile Wahrnehmbarkeit zu gewährleisten, ist das Funktionselement formstabil im Sinne von jedenfalls weniger formnachgiebig als der Pumpabschnitt gestaltet. Der Pumpabschnitt ist vorzugsweise weichelastisch formnachgiebig. Das Funktionselement ist vorzugsweise formstabil. Sofern die Tropfkammeranordnung eine mehrteilige Gestaltung des Gehäuses aufweist, ist der Pumpabschnitt vorzugsweise einem Tropfkammerunterteil des Gehäuses zugeordnet. Der Pumpabschnitt umgrenzt ein Teilvolumen der Tropfkammer. Das Funktionselement kann hierbei stets in dem Teilvolumen angeordnet sein oder erst infolge eines Aufschwimmens auf der medizinischen Flüssigkeit in das Teilvolumen und damit in den Pumpabschnitt hinein verlagert sein. Bei einem gegenüber dem angestrebten Flüssigkeitspegel niedrigeren Flüssigkeitspegel ist das Funktionselement demgegenüber taktil nicht wahrnehmbar. "Taktil nicht wahrnehmbar" schließt dabei auch eine im Vergleich zu der Pegelschwimmposition ihrem Umfang nach geringere taktile Wahrnehmbarkeit ein. Demgemäß muss das Funktionselement in der wenigstens einen weiteren Schwimmposition nicht vollständig taktil nicht wahrnehmbar sein, was jedoch vorteilhaft sein kann.

Weiter gemäß der Erfindung ist die radiale Formnachgiebigkeit des Pumpabschnitts in der Pegelschwimmposition mittels des Funktionselements verringert, vorzugsweise wenigstens im Wesentlichen vollständig blockiert. Durch diese Ausgestaltung der Erfindung wird einem initialen Befüllen der Tropfkammer über den angestrebten Flüssigkeitspegel hinweg besonders zuverlässig entgegengewirkt. Sobald das Funktionselement die Pegelschwimmposition und damit der Flüssigkeitspegel die angestrebte Höhe erreicht, verringert das Funktionselement die radiale Formnachgiebigkeit des Pumpabschnitts und beeinträchtigt somit die Pumpbeweglichkeit. Eine nochmals erhöhte Patientensicherheit kann erreicht werden, wenn das Funktionselement die Pumpbeweglichkeit des Pumpabschnitts in der Pegelschwimmposition wenigstens im Wesentlichen vollständig, vorzugsweise vollständig, blockiert. Um eine entsprechende Verringerung oder gar Blockierung der Pumpbeweglichkeit zu erreichen, sind ein radialer Außendurchmesser des Funktionselements und ein radialer Innendurchmesser des Pumpabschnitts im Ruhezustand dementsprechend aufeinander abgestimmt.

In weiterer Ausgestaltung der Erfindung beträgt ein radialer Außendurchmesser des Funktionselements mehr als 75 %, bevorzugt mehr als 85 %, und besonders bevorzugt mehr als 95 %, eines Innendurchmessers des Pumpabschnitts im Ruhezustand. Eine für die meisten Anwendungsfälle ausreichende Verringerung der Pumpbeweglichkeit kann bereits erreicht werden, wenn der Außendurchmesser 25 % des Innendurchmessers beträgt. Eine hierzu deutlichere Verringerung kann erreicht werden, wenn der Außendurchmesser mehr als 85 % des Innendurchmessers beträgt. Eine wenigstens im Wesentlichen vollständige Blockierung der Pumpbeweglichkeit kann erreicht werden, wenn der Außendurchmesser mehr als 95 % des Innendurchmessers beträgt.

In weiterer Ausgestaltung der Erfindung ist das Funktionselement in Form einer Scheibe, einer Kugel, eines Tropfens oder eines Polyeders, vorzugsweise eines Oktaeders, ausgebildet. Grundsätzlich ist jedwede Formgebung des Funktionselements zweckmäßig, die zum einen eine anforderungsgerechte visuelle Wahrnehmbarkeit zur Kontrolle des Flüssigkeitspegels und zum anderen eine anforderungsgerechte taktile Wahrnehmbarkeit zur Rückmeldung des Flüssigkeitspegels bei einer Betätigung des Pumpabschnitts gewährleistet. Dabei hat sich eine scheiben-, kugel-, tropfen- oder polyederförmige Gestaltung als besonders vorteilhaft erwiesen. Sofern das Funktionselement in Form eines Oktaeders ausgebildet ist, ergibt sich eine besonders vorteilhafte taktile Wahrnehmbarkeit bei gleichzeitig anforderungsgerechter visueller Wahrnehmbarkeit des Funktionselements.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematisch stark vereinfachter und teilweise abgeschnittener Darstellung ein medizinisches Infusionssystem mit einer Ausführungsform einer erfindungsgemäßen Tropfkammeranordnung,
- Fig. 2: in abgeschnittener und teilweise aufgeschnittener vergrößerter Detaildarstellung die Tropfkammeranordnung nach Fig. 1,
- Fig. 3: in einer der Fig. 2 entsprechenden Darstellungsweise die Tropfkammeranordnung nach den Fig. 1 und 2 in einem bis zu einem angestrebten Flüssigkeitspegel mit einer medizinischer Flüssigkeit befüllten Zustand und
- Fig. 4, 5, 6: jeweils in schematisch stark vereinfachter Darstellung alternative Ausgestaltungen eines in einer Tropfkammer der Tropfkammeranordnung nach den Fig. 1 bis 3 beweglich angeordneten Funktionselements.

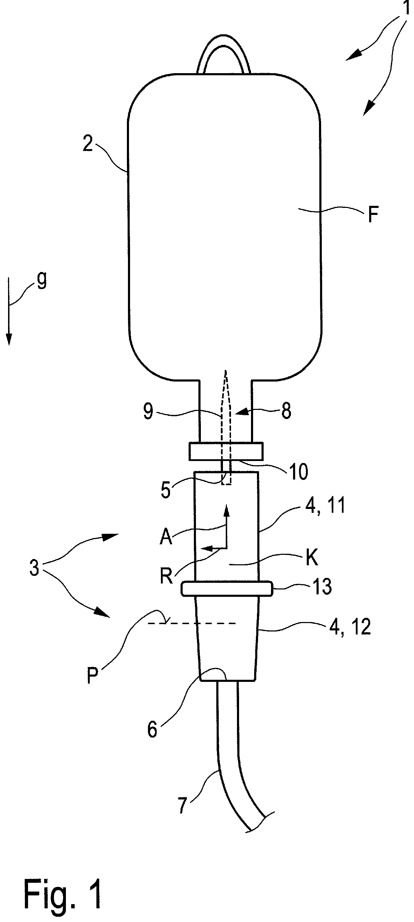

Gemäß Fig. 1 ist ein medizinisches Infusionssystem 1, das auch als Uberleitungssystem 1 bezeichnet werden kann, zur Verwendung bei einer schwerkraftgetriebenen Infusionstherapie vorgesehen.

Das Infusionssystem 1 weist vorliegend einen Infusionsbehälter 2 auf, der mit einer nicht näher bezeichneten und im Rahmen der Infusionstherapie einem Patienten zu verabreichenden medizinischen Flüssigkeit F befüllt ist. Der Infusionsbehälter ist vorliegend in Form eines Infusionsbeutels 2 ausgebildet und weist insoweit eine allgemein bekannte Funktionsweise und Gestaltung auf, so dass auf weitere Details des Infusionsbeutels 2 an dieser Stelle nicht weiter eingegangen werden braucht.

Das Infusionssystem 1 weist zudem eine erfindungsgemäße Tropfkammeranordnung 3 mit einem Gehäuse 4 auf. Das Gehäuse 4 umgrenzt eine Tropfkammer K und weist einen Einlass 5 und einen Auslass 6 auf. Der Einlass 5 ist zum Einleiten der medizinischen Flüssigkeit F in die Tropfkammer K vorgesehen. Der Auslass 6 ist demgegenüber zum Ableiten der medizinischen Flüssigkeit F aus der Tropfkammer K vorgesehen und ist zu diesem Zweck auf grundsätzlich bekannte Weise fluidleitend mit einer der Tropfkammeranordnung 3 nachgeordneten Schlauchleitung 7 verbunden. Die Schlauchleitung 7 weist an ihrem zeichnerisch nicht dargestellten und der Tropfkammeranordnung 3 gegenüberliegenden Ende einen Konnektor auf, der insbesondere zur fluidleitenden Verbindung mit einem patientenseitigen Zugang oder einem anderen fluidleitenden System vorgesehen sein kann. Die Tropfkammeranordnung 3 ist einlassseitig fluidleitend mit einem Einstechteil 8 verbunden, das einen grundsätzlich bekannten Aufbau und eine als solche bekannte Funktionsweise aufweist. Das Einstechteil 8 ist vorliegend unmittelbar mit dem Gehäuse 4 der Tropfkammeranordnung 3 verbunden und kann insbesondere einstückig mit diesem ausgebildet sein. Das Einstechteil 8 weist einen hohlen Einstechdorn 9 auf, durch den sich ein einends in den Einlass 5 mündendes nicht näher ersichtliches Lumen erstreckt.

Zur Überleitung der medizinischen Flüssigkeit F von dem Infusionsbeutel 2 in den besagten patientenseitigen Zugang oder das besagte fluidleitende System wird die Tropfkammeranordnung 3 zusammen mit dem Einstechteil 8 ausgehend von einer zeichnerisch nicht dargestellten und nicht mit dem Infusionsbeutel 2 verbundenen Konfiguration mit dem Einstechdorn 9 voran in Axialrichtung A der Tropfkammer K in einen Einstechabschnitt 10 des Infusionsbeutels 2 eingestochen. Hierdurch nimmt das Infusionssystem 1 die anhand Fig. 1 ersichtliche Konfiguration ein. Der Infusionsbeutel 2 ist hierbei auf grundsätzlich bekannte Weise an einem nicht dargestellten Infusionsständer oder dergleichen vertikal nach unten hängend befestigt. Das heißt in der anhand Fig. 1 ersichtlichen Konfiguration ist die Axialrichtung A parallel zu einem Erdschwerevektor g erstreckt. In dieser Konfiguration kann die medizinische Flüssigkeit F über das Lumen des Einstechdorns 9 zum Einlass 5 der Tropfkammer K gelangen und von dort tropfenweise in die Tropfkammer K fallen. Hierbei stellt sich auf grundsätzlich bekannte Weise ein Flüssigkeitspegel P der medizinischen Flüssigkeit F in der Tropfkammer K ein. Das heißt die Tropfkammer K ist in Axialrichtung A bis zu einer gewissen Höhe mit medizinischem Fluid befüllt. Oberhalb des Flüssigkeitspegels P und unterhalb des Einlasses 5 ist ein nicht näher bezeichnetes Luftpolster bzw. ein Luftraum ausgebildet, durch den die Tropfen der medizinischen Flüssigkeit F schwerkraftbedingt hindurchfallen.

Zur visuellen Kontrolle des Flüssigkeitspegels P ist das Gehäuse 4 wenigstes abschnittsweise blickdurchlässig ausgebildet. Vorliegend ist das Gehäuse 4 zweiteilig gestaltet und weist insoweit ein Tropfkammeroberteil 11 und ein Tropfkammerunterteil 12 auf, was jedoch nicht zwingend ist. Stattdessen kann das Gehäuse 4 auch ein- oder mehr als zweiteilig ausgeführt sein. Das Tropfkammeroberteil 11 und das Tropfkammerunterteil 12 sind vorliegend jeweils aus Kunststoff gefertigt und zur Herstellung einer fluiddichten Fügeverbindung an ihren einander gegenüberliegenden Stirnendbereichen unter Ausbildung eines Radialbunds 13 mit Kunststoff umspritzt. Auch dies ist nicht zwingend. Bei einer zeichnerisch nicht dargestellten Ausführungsform können das Tropfkammeroberteil 11 und das Tropfkammerunterteil 12 ohne Ausbildung eines Radialbunds ineinander eingesteckt oder stumpf aufeinandergefügt sein.

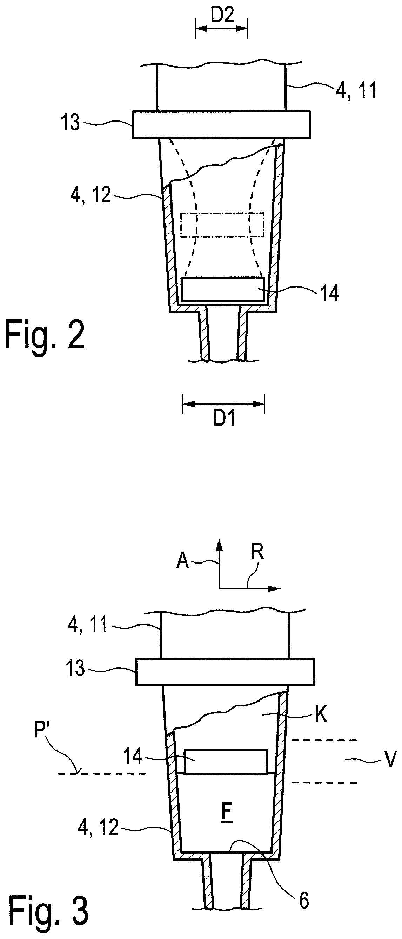

Zur verbesserten visuellen Kontrolle des Flüssigkeitspegels P ist erfindungsgemäß ein in der Tropfkammer K beweglich angeordnetes Funktionselement 14 vorgesehen (vgl. Fig. 2, 3). Das Funktionselement 14 ist auf der medizinischen Flüssigkeit F schwimmfähig ausgebildet. Dementsprechend ist eine Dichte des Funktionselements 14 auf eine Dichte der medizinischen Flüssigkeit F abgestimmt und geringer als diese. Das Funktionselement 14 ist in Abhängigkeit des Flüssigkeitspegels P in Axialrichtung A der Tropfkammer zwischen unterschiedlichen Schwimmpositionen verlagerbar. Anhand Fig. 2 ist das Funktionselement 14 in einem nicht mit medizinischer Flüssigkeit F befüllten Zustand der Tropfkammer K gezeigt, wobei die dort eingenommene Position des Funktionselements 14 dessen ungeachtet auch als eine Schwimmposition bezeichnet werden soll. Anhand Fig. 3 ist das Funktionselement 14 in einer Pegelschwimmposition gezeigt, die einem in der Tropfkammer K angestrebten Flüssigkeitspegel P' zugeordnet ist. Der angestrebte Flüssigkeitspegel P' kann auch als Soll-Flüssigkeitspegel bezeichnet werden. Der angestrebte Flüssigkeitspegel P' ist aus medizinischer Sicht weder zu hoch noch zu gering. Bei einer Befüllung der Tropfkammer K mit dem angestrebten Flüssigkeitspegel P ist zum einen ein ausreichendes Luftpolster zwischen dem Einlass 5 und dem Auslass 6 gewährleistet, was einer Bildung sogenannter Keimstraßen entgegenwirkt. Zum anderen ist sichergestellt, dass die medizinische Flüssigkeit F nicht direkt ausgehend von dem Einlass 5 in den Auslass 6 einfällt, was zu einem unerwünschten Eintrag von Luft in die Schlauchleitung 7 führen könnte. Zur visuellen Kontrolle des Flüssigkeitspegels ist das Funktionselement 14 wenigstens in der anhand Fig. 3 ersichtlichen Pegelschwimmposition durch das wenigstens abschnittsweise blickdurchlässige Gehäuse 4 hindurch einsehbar. Um diese Einsehbarkeit des Funktionselements 14 zu gewährleisten, ist das Gehäuse 4 wenigstens in einem dem angestrebten Flüssigkeitspegel P' zugeordneten Gehäuseabschnitt V (vgl. Fig. 3) blickdurchlässig ausgebildet. Vorliegend ist das Gehäuse 4 nicht lediglich in dem Gehäuseabschnitt V, sondern stattdessen im Wesentlichen vollständig blickdurchlässig ausgebildet. Dementsprechend ist vorliegend sowohl das Tropfkammerunterteil 12 als auch das Tropfkammeroberteil 11 aus blickdurchlässigem Kunststoff gefertigt.

Um eine möglichst hohe Auftriebskraft und damit ein leichtgängiges Aufschwimmen des Funktionselements 14 zu gewährleisten, weist das Funktionselement 14 vorliegend eine Dichte auf, die kleiner als 0,10 g/cm³ ist. Dies ist jedoch nicht zwingend. So kann bereits eine Dichte kleiner als 1,05 g/cm³ für viele medizinische Anwendungsfälle bzw. eine Vielzahl relevanter medizinischer Flüssigkeiten ausreichend sein. Eine im praktischen Sinne uneingeschränkte Schwimmfähigkeit des Funktionselements 14 kann bereits bei einer Dichte von 0,50 g/cm³ erreicht sein.

Zum initialen Befüllen der Tropfkammer K mit der medizinischen Flüssigkeit F weist das Gehäuse 4 einen Pumpabschnitt auf, der vorliegend durch das Tropfkammerunterteil 12 gebildet ist. Das Tropfkammerunterteil 12 ist in Radialrichtung R der Tropfkammer K elastisch formnachgiebig ausgebildet. Zum initialen Ansaugen der medizinischen Flüssigkeit F durch den Einlass 5 in die Tropfkammer K kann das Tropfkammerunterteil 12 mittels einer manuellen radialen Pumpbewegung zwischen einem in Radialrichtung eingedrückten Pumpzustand und einem nicht manuell eingedrückten Ruhezustand überführt werden. Dies ist anhand Fig. 2 verdeutlicht, wobei der radial eingedrückte Pumpzustand des Tropfkammerunterteils 12 durch die dort vorhandenen strichlierten Linien verdeutlicht ist. Der Ruhezustand des Tropfkammerunterteils 12 bezieht sich auf die in erster Linie erkennbare Schnittdarstellung der Fig. 2. Um diese Pumpfunktion zu gewährleisten, ist das Tropfkammerunterteil 12 vorliegend aus einem weichelastischen Kunststoff gefertigt.

Zum initialen Befüllen der Tropfkammer K mit der medizinischen Flüssigkeit F wird das Tropfkammerunterteil 12 in einem in den Infusionsbeutel 2 eingestochenen Zustand des Einstechteils 8 in Axialrichtung A in etwa mittig zwischen dem Auslass 6 und dem Radialbund 13 zwischen Daumen und Zeigefinger einer Hand gegriffen und wiederholt radial eingedrückt. Hierdurch gelangt die medizinische Flüssigkeit F auf die vorbeschriebene Weise unterdruckbedingt in die Tropfkammer K, wodurch der Flüssigkeitspegel ausgehend von dem anhand Fig. 2 ersichtlichen unbefüllten Zustand in Richtung des angestrebten Flüssigkeitspegels P' (vgl. Fig. 3) ansteigt.

Um ein übermäßiges manuelles Pumpen und damit ein Überfüllen der Tropfkammer K über den angestrebten Flüssigkeitspegel P' hinaus zu vermeiden, ist das Funktionselement 14 im Vergleich zu dem Tropfkammerunterteil 12 formstabil ausgebildet und in der Pegelschwimmposition, d.h. bei Erreichen des angestrebten Flüssigkeitspegels P', im eingedrückten Pumpzustand durch den Pumpabschnitt 12 hindurch taktil wahrnehmbar. Dies ist anhand Fig. 2 verdeutlicht. Die dort gezeigte strichpunktierte Umrisslinie lässt das Funktionselement 14 in der Pegelschwimmposition erkennen. Infolge der formstabilen Gestaltung des Funktionselements 14 ist das als solche elastisch form nachgiebige Tropfkammerunterteil 12 nur noch in verringertem Maße radial in Richtung des Pumpzustands eindrückbar. Hierdurch erhält ein das Infusionssystem 2 handhabendes Personal eine unmittelbare Rückmeldung, sofern beim initialen Befüllen der Tropfkammer K eine Überfüllung droht. Demgegenüber ist das Funktionselement 14 der weiteren anhand Fig. 2 ersichtlichen Schwimmposition, die einem niedrigeren Flüssigkeitspegel bzw. einem nicht gefüllten Zustand der Tropfkammer K zugeordnet ist, taktil nicht oder jedenfalls in einem geringeren Maße taktil wahrnehmbar.

Vorliegend ist ein radialer Außendurchmesser D1 des Funktionselements 14 derart auf einen radialen Innendurchmesser D2 des Tropfkammerunterteils 12 im Pumpzustand abgestimmt, dass die Pumpbeweglichkeit des Tropfkammerunterteils 12 in der Pegelschwimmposition im Wesentlichen vollständig blockiert ist. Der Außendurchmesser D1 des Funktionselements 14 ist hierbei lediglich geringfügig geringer als ein nicht näher bezeichneter Innendurchmesser des Tropfkammerunterteils 12 im Ruhezustand.

Das Gehäuse 4 weist vorliegend eine hohlzylindrische Grundform mit einer kreisringförmigen Axialquerschnittsfläche auf. Das Funktionselement 14 der Ausführungsform nach den Fig. 1 bis 3 weist eine kreisscheibenförmige Gestaltung auf und ist dementsprechend in Form einer Scheibe ausgebildet. Eine solche Formgebung des Funktionselements 14 ist jedoch nicht zwingend.

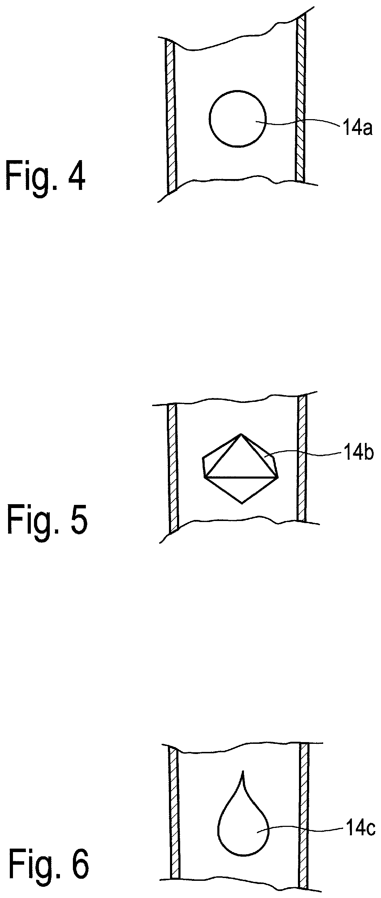

Alternative Formgebungen des Funktionselements sind anhand der Fig. 4 bis 6 verdeutlicht. So zeigt Fig. 4 ein Funktionselement 14a in Form einer Kugel. Eine weitere alternative Ausgestaltung nach Fig. 5 sieht ein Funktionselement 14b in Form eines Polyeders vor, der vorliegend als Oktaeder gestaltet ist. Anhand Fig. 6 ist ein Funktionselement 14c in Form eines Tropfens ersichtlich. Die anhand der Fig. 4 bis 6 ersichtlichen Formgebungen sind rein exemplarisch, wobei insbesondere eine maßliche Abstimmung zwischen den Funktionselementen 14a bis 14c und den in den Fig. 4 bis 6 jeweils ersichtlichen Gehäuseabschnitten zeichnerisch nicht maßstabsgetreu verdeutlicht ist.

Bei einer zeichnerisch nicht dargestellten Ausführungsform kann das Funktionselement lediglich zur visuellen Kontrolle des Pegelstands P vorgesehen sein. Insoweit sieht diese Ausführungsform keine taktile Wahrnehmbarkeit des Funktionselements in der Pegelschwimmposition zur verbesserten Kontrolle des Flüssigkeitspegels P beim initialen Befüllen der Tropfkammer K vor. Anders ausgedrückt ist die bei der anhand der Fig. 1 bis 3 verdeutlichte Merkmalskombination nicht zwingend, sondern das Funktionselement kann hiervon abweichend ausschließlich zur visuellen Kontrolle vorgesehen sein. Bei einer weiteren zeichnerisch nicht dargestellten Ausführungsform ist das Funktionselement demgegenüber allein zur taktilen Wahrnehmbarkeit in der Pegelschwimmposition vorgesehen. Bei dieser Ausgestaltung der Erfindung ist insbesondere eine abschnittsweise blickdurchlässige Gestaltung des Gehäuses und/oder ein Pegelindikator, der dem Gehäuse zugeordnet und zur Kontrolle eines Flüssigkeitspegels der medizinischen Flüssigkeit in der Tropfkammer vorgesehen ist, nicht zwingend notwendig.

## Patentansprüche

1. Tropfkammeranordnung (3) für ein medizinisches Infusionssystem (1), aufweisend ein Gehäuse (4, 11, 12), das wenigstens abschnittsweise blickdurchlässig ausgebildet ist und eine Tropfkammer (K) zur Aufnahme einer medizinischen Flüssigkeit (F) umgrenzt, einen Einlass (5), der an dem Gehäuse (4, 11) angeordnet und zum Einleiten der medizinischen Flüssigkeit (F) in die Tropfkammer (K) vorgesehen ist, einen Auslass (6), der an dem Gehäuse (4, 12) angeordnet und zum Ableiten der medizinischen Flüssigkeit (F) aus der Tropfkammer (K) vorgesehen ist, und aufweisend einen Pegelindikator, der dem Gehäuse (4, 11, 12) zugeordnet und zur visuellen Kontrolle eines Flüssigkeitspegels (P) der medizinischen Flüssigkeit (F) in der Tropfkammer (K) vorgesehen ist, wobei der Pegelindikator ein in der Tropfkammer beweglich angeordnetes Funktionselement (14, 14a bis 14c) ist, das auf der medizinischen Flüssigkeit (F) schwimmfähig ausgebildet ist und in Abhängigkeit des Flüssigkeitspegels (P) in Axialrichtung (A) der Tropfkammer (K) zwischen unterschiedlichen Schwimmpositionen verlagerbar ist und wenigstens in einer Pegelschwimmposition, die einem angestrebten Flüssigkeitspegel (P') zugeordnet ist, zur Kontrolle des Flüssigkeitspegels (P) durch das wenigstens abschnittsweise blickdurchlässige Gehäuse (4, 11, 12) hindurch einsehbar ist, wobei das Gehäuse (4, 11, 12) einen Pumpabschnitt (12) aufweist, der in Radialrichtung (R) der Tropfkammer (K) elastisch formnachgiebig ausgebildet und in Axialrichtung (A) der Tropfkammer (K) zwischen dem Einlass (5) und dem Auslass (6) angeordnet ist, wobei der Pumpabschnitt (12) zum initialen Ansaugen der medizinischen Flüssigkeit (F) durch den Einlass (5) mittels einer manuellen radialen Pumpbewegung zwischen einem in Radialrichtung (R) eingedrückten Pumpzustand und einem nicht manuell eingedrückten Ruhezustand überführbar ist, **dadurch gekennzeichnet, dass** das Funktionselement (14, 14a bis 14c) im Vergleich zu dem Pumpabschnitt formstabil ausgebildet und in der Pegelschwimmposition im eingedrückten Pumpzustand des Pumpabschnitts (12) durch den Pumpabschnitt (12) hindurch taktil wahrnehmbar und in wenigstens einer weiteren Schwimmposition, die einem niedrigeren Flüssigkeitspegel zugeordnet ist, taktil nicht wahrnehmbar ist, und wobei die radiale Formnachgiebigkeit des Pumpabschnitts (12) in der Pegelschwimmposition mittels des Funktionselements (14, 14a bis 14c) verringert, vorzugsweise wenigstens im Wesentlichen vollständig blockiert, ist.

2. Tropfkammeranordnung (3) nach Anspruch 1, **dadurch gekennzeichnet, dass** das Funktionselement (14, 14a bis 14c) in allen Schwimmpositionen einsehbar ist.

3. Tropfkammeranordnung (3) nach Anspruch 1 oder 2, **dadurch gekennzeichnet, dass** das Funktionselement (14, 14a bis 14c) eine Dichte aufweist, die kleiner als 1,05 g/cm³, bevorzugt kleiner als 0,50 g/cm³, und besonders bevorzugt kleiner als 0,10 g/cm³ ist.

4. Tropfkammeranordnung (3) nach einem der vorhergehenden Ansprüche, **dadurch gekennzeichnet, dass** ein radialer Außendurchmesser (D1) des Funktionselements (14, 14a bis 14c) mehr als 75 %, bevorzugt mehr als 85 %, und besonders bevorzugt mehr als 95 %, eines Innendurchmessers des Pumpabschnitts (12) im Ruhezustand beträgt.

5. Tropfkammeranordnung (3) nach einem der vorhergehenden Ansprüche, **dadurch gekennzeichnet, dass** das Funktionselement in Form einer Scheibe (14), einer Kugel (14a), eines Tropfens (14c) oder eines Polyeders (14b), vorzugsweise eines Oktaeders, ausgebildet ist.

## Claims

1. Drip chamber assembly (3) for a medical infusion system (1), having a housing (4, 11, 12), which is at least partially transparent and delimits a drip chamber (K) for receiving a medical liquid (F), an inlet (5), which is arranged on the housing (4, 11) and is provided for the introduction of the medical liquid (F) into the drip chamber (K), and an outlet (6), which is arranged on the housing (4, 12) and is provided for the discharging of the medical liquid (F) from the drip chamber (K), and having a level indicator, which is assigned to the housing (4, 11, 12) and is provided to allow visual monitoring of a liquid level (P) of the medical liquid (F) in the drip chamber (K), wherein the level indicator is a functional element (14, 14a to 14c) which is arranged movably in the drip chamber and which is designed to float on the medical liquid (F) and, depending on the liquid level (P), is displaceable in the axial direction (A) of the drip chamber (K) between different floating positions and, at least in one floating position which is assigned to a desired liquid level (P'), can be seen through the at least partially transparent housing (4, 11, 12) in order to allow monitoring of the liquid level (P), wherein the housing (4, 11, 12) has a pump section (12) which is elastically resilient in the radial direction (R) of the drip chamber (K) and is arranged in the axial direction (A) of the drip chamber (K) between the inlet (5) and the outlet (6), wherein the pump section (12), for the initial suction of the medical liquid (F) through the inlet (5), can be transferred, by means of a manual radial pumping movement, between a pump state in which it is pressed inwards in the radial direction (R) and a resting state in which it is not manually pressed inwards, **characterized in that** the functional element (14, 14a to 14c) is dimensionally stable compared to the pump section and, in the floating position in the inwardly pressed state of the pump section (12), is perceptible to touch through the pump section (12), and, in at least one further floating position, which is assigned to a lower liquid level, is not perceptible to touch, and wherein the radial resiliency of the pump section (12) in the floating position is reduced by means of the functional element (14, 14a to 14c), preferably at least substantially completely blocked.

2. Drip chamber assembly (3) according to Claim 1, **characterized in that** the functional element (14, 14a to 14c) is visible in all floating positions.

3. Drip chamber assembly (3) according to Claim 1 or 2, **characterized in that** the functional element (14, 14a to 14c) has a density of less than 1.05 g/cm³, preferably less than 0.50 g/cm³, and particularly preferably less than 0.10 g/cm³.

4. Drip chamber assembly (3) according to any one of the preceding claims, **characterized in that** a radial outer diameter (D1) of the functional element (14, 14a to 14c) is more than 75%, preferably more than 85%, and particularly preferably more than 95%, of an inner diameter of the pump section (12) in the resting state.

5. Drip chamber assembly (3) according to any one of the preceding claims, **characterized in that** the functional element is in the form of a disc (14), a sphere (14a), a drop (14c) or a polyhedron (14b), preferably an octahedron.

## Revendications

1. Ensemble formant chambre de goutte-à-goutte (3) pour un système de perfusion médical (1), comportant un boîtier (4, 11, 12) qui est formé de manière à être au moins par sections transparent et qui définit une chambre de goutte-à-goutte (K) destinée à recevoir un liquide médical (F), une entrée (5), qui est disposée sur le boîtier (4, 11) et est prévue pour acheminer le liquide médical (F) dans la chambre de goutte-à-goutte (K), une sortie (6), qui est disposée sur le boîtier (4, 12) et est prévue pour évacuer le liquide médical (F) hors de la chambre de goutte-à-goutte (K), et comportant un indicateur de niveau, qui est associé au boîtier (4, 11, 12) et est prévu pour surveiller visuellement un niveau de liquide (P) du liquide médical (F) dans la chambre de goutte-à-goutte (K), l'indicateur de niveau étant un élément fonctionnel (14, 14a à 14c) disposé de manière mobile dans la chambre de goutte-à-goutte, qui peut flotter sur le liquide médical (F) et peut être déplacé entre différentes positions de flottaison dans la direction axiale (A) de la chambre de goutte-à-goutte (K) en fonction du niveau de liquide (P) et est visible à travers le boîtier (4, 11, 12) au moins par sections transparent au moins dans une position de flottaison de niveau, qui est associée à un niveau de liquide (P') souhaité, pour surveiller le niveau de liquide (P), le boîtier (4, 11, 12) comportant une section de pompage (12), qui est formée de manière à se déformer élastiquement dans la direction radiale (R) de la chambre de goutte-à-goutte (K) et qui est disposée entre l'entrée (5) et la sortie (6) dans la direction axiale (A) de la chambre de goutte-à-goutte (K), la section de pompage (12) pouvant être transférée entre un état de pompage enfoncé dans la direction radiale (R) et un état de repos non enfoncé manuellement au moyen d'un déplacement de pompage radial manuel pour l'aspiration initiale du liquide médical (F) à travers l'entrée (5), **caractérisé en ce que** l'élément fonctionnel (14, 14a à 14c) présente une forme indéformable en comparaison avec la section de pompage et est perceptible au toucher à travers la section de pompage (12) dans la position de flottaison de niveau dans l'état de pompage enfoncé de la section de pompage (12), et n'est pas perceptible au toucher dans au moins une autre position de flottaison, qui est associée à un niveau de liquide inférieur, et l'élément fonctionnel (14, 14a à 14c) permettant de réduire, de préférence de bloquer au moins sensiblement complètement, la déformation radiale de la section de pompage (12) dans la position de flottaison de niveau.

2. Ensemble formant chambre de goutte-à-goutte (3) selon la revendication 1, **caractérisé en ce que** l'élément fonctionnel (14, 14a à 14c) est visible dans toutes les positions de flottaison.

3. Ensemble formant chambre de goutte-à-goutte (3) selon la revendication 1 ou 2, **caractérisé en ce que** l'élément fonctionnel (14, 14a à 14c) présente une densité qui est inférieure à 1,05 g/cm³, de manière préférée est inférieure à 0,50 g/cm³, et de manière particulièrement préférée inférieure à 0,10 g/cm³.

4. Ensemble formant chambre de goutte-à-goutte (3) selon l'une des revendications précédentes, **caractérisé en ce qu'**un diamètre extérieur radial (D1) de l'élément fonctionnel (14, 14a à 14c) représente plus de 75 %, de manière préférée plus de 85 %, et de manière particulièrement préférée plus de 95 %, d'un diamètre intérieur de la section de pompage (12) dans l'état de repos.

5. Ensemble formant chambre de goutte-à-goutte (3) selon l'une des revendications précédentes, **caractérisé en ce que** l'élément fonctionnel est réalisé sous la forme d'un disque (14), d'une sphère (14a), d'une goutte (14c) ou d'un polyèdre (14b), de préférence d'un octaèdre.
